# EUROPEAN PATENT SPECIFICATION

(11) **EP 1 151 735 B1**
(45) Date of publication and mention of the grant of the patent: **05.10.2005**
(21) Application number: 01304069.6
(22) Date of filing: 04.05.2001
(51) Int. Cl.: A61F 13/534

(54) **Absorbent core for use in a sanitary absorbent article and method for manufacturing**
Absorbierende Kernschicht zur Verwendung in einem absorbierenden Hygieneartikel und Verfahren zur Herstellung
Noyau absorbant utilisé dans un article absorbant hygiénique et son procédé de fabrication

(30) Priority: 05.05.2000 BR 0002299
(43) Date of publication of application: 07.11.2001
(73) Proprietor: Johnson & Johnson Industrial Ltda., Sao Jose dos Campos, SP (BR)
(72) Inventor: Proglhof, Igor Philip Passos, Sao Jose dos Campos SP (BR)
(74) Representative: Mercer, Christopher Paul

(56) References cited:
- EP-A- 0 649 644
- EP-A- 0 700 672
- EP-A- 0 920 846
- WO-A-99/42067
- GB-A- 2 014 046
- US-A- 4 551 191
- US-A- 4 560 379
- US-A- 4 596 567
- US-A- 4 960 477
- US-A- 5 947 945

## Description

### FIELD OF THE INVENTION

The present invention relates to an absorbent core for use in a disposable sanitary absorbent articles such as panty liners, sanitary napkins and adult incontinence devices that are designed and adapted to be worn in a crotch portion of a wearer's undergarment and to receive and contain menses and other vaginal discharges.

### BACKGROUND OF THE INVENTION

Sanitary absorbent articles are used to collect and contain vaginal exudation, especially inter-menstrual secretions, menstrual blood and also urine in the cases of incontinence. To improve comfort, such articles avoid the use of thick layers of absorbing material.

The sanitary absorbent articles known in the art are typically composed of a body made of absorbing material, having a substantially planar and elongated shape, which is enclosed by an upper pervious layer adaptive to contact the user's body, and by a lower impervious layer co-operating with her panty.

The upper pervious layer is adaptive to contact the user's pelvic region and, therefore, is made of material as complaisant and non-irritating as possible. According to the present state of the art, this layer can be a perforated plastic film, a porous or crosslinked foam, a sheet of a woven or non-woven material with natural fibers (wood or cotton fibers), artificial fibers (polyester or polypropylene), or even a combination of synthetic or natural fibers. Said upper layer can be of hydrophobic material in order to present a trend to remain dry.

Said lower impervious layer, on the other hand, has the function of preventing the fluid absorbed and retained in the absorbing body from passing to the user's clothing or skin, being optionally made from a sheet of polyethylene. Said lower layer can be vapor permeable and, in this case, it is provided with small pores or it is made from an impervious to liquid, non-woven material.

The body made from absorbing material basically comprises two elements: a transfer layer and an absorbing core. Said absorbing core, in turn, is comprised of an absorption sheet enclosing a superabsorbent material.

Typically, the material used in the manufacture of the absorption sheet is a paper known as "air laid". This kind of paper is made from cellulose and presents structure in non-oriented fibers, thus providing a good capacity of liquid distribution. Still some kinds of non-fabric in substitution to the paper "air laid" are used.

The superabsorbent material is known in the art and normally it is presented in powder, in the form of hygroscopic granules. Such granules are stuck to the absorption sheet and they have the function of absorbing and retaining the liquid within the absorbing core of the sanitary absorbent article.

The material used for manufacturing the absorption sheet of the absorbing core is particularly important so that the absorbent has good efficiency, since this sheet must be capable of absorbing, distributing and transferring the liquid to the superabsorbent material uniformly.

A material known in the art, which presents features similar to those set forth above is described in the document EP0146190. Such material has multiple absorbing layers, which enclose a superabsorbent, thereby forming a structure that goes through an embossing process.

One of the problems found in the manufacture of sanitary absorbent articles is that the paper type "air laid" has high cost, rising the final value of the respective absorbent.

### SUMMARY OF THE INVENTION

According to the present invention there is provided an absorbent article as claimed in appendant claims 1 to 8, an absorbent core for use in a sanitary article as claimed in appendant claims 9 to 12 and a method for the manufacture of an absorbent core as claimed in claims 13 and 14. The core of the sanitary absorbent article utilizes "wet laid" paper in its manufacture of an absorbing core, as a substitute for the "air laid" paper presently used in the manufacture of said absorbing core.

### BRIEF DESCRIPTION OF THE DRAWINGS

While the specification concludes with claims particularly pointing out and distinctly claiming the present invention, it is believed that the present invention will be better understood from the following description in conjunction with the following drawings, in which like reference numbers identify identical elements and wherein:
Figure 1 represents a perspective view of the upper face of a sanitary absorbent article according to this invention;
Figure 2 represents a sectional view of the article illustrated in Figure 1;
Figure 3 represents a perspective view of a lower face of the absorbing core according to this invention;
Figure 4 represents an schematic view of the type of the embossing used in manufacturing of the absorbing core illustrated in Figure 3;
Figure 5 represents an upper perspective view of the absorbent sheet according to this invention;
Figure 6 an enlargement of the absorbent sheet shown in Figure 5.

### DETAILED DESCRIPTION OF THE INVENTION

As already known in the art, a sanitary absorbent article comprises a body of absorbing material 5 of substantially planar shape, enclosed by an upper layer pervious to liquid 2, suitable to intimate contact with the user's pelvic region and by a lower impervious layer 3, which can cooperate with the panty (not shown).

Still can be envisaged flexible side tabs 4 which extend laterally as an extension of the upper 2 and lower 3 layers, or one of them only, or still separated from these layers, but associated to the absorbent 1. Each of the lower faces of these tabs 4 can still have a region of adhesive (not shown), allowing its fastening to the crotch portion of the user's panty. Another adhesive region (also not shown) can be envisaged in a longitudinal center portion of the lower layer 3 of the absorbent 1.

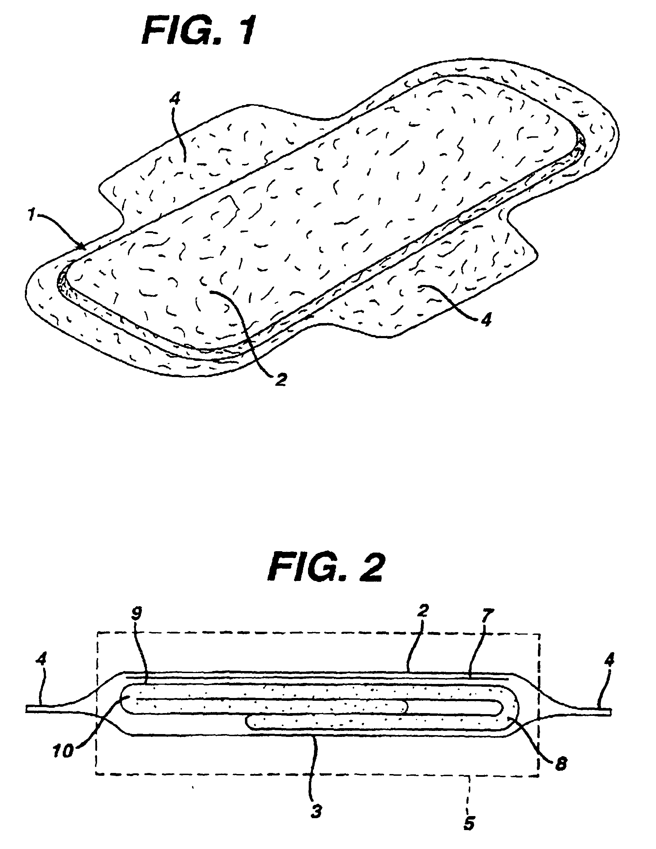

As illustrated in figure 2, the absorbing body 5 (delimited by the dashed area) is made up of a transfer layer 7 adjacent to an absorbing core 8 which, in turn, is comprised of an absorption sheet 9 having its inner surface covered with superabsorbent material 10.

According to this invention, the absorption sheet 9 of the absorbing core 8 shall be manufactured in paper type "wet laid", replacing the paper "air laid" of a conventional sanitary absorbent article.

As known in the art, the paper "wet laid" or "water laid", or even paper "tissue", is manufactured by cellulose agglutination on a pervious screen, providing thus a paper layer which will be pressed thereby obtaining a fine, absorbing paper.

However, the simple change of paper "air laid", which is currently used in the manufacture of the absorption sheet 9, by the paper type "wet laid" is not possible, since the paper "wet laid" presents a series of technical problems which will be solved according to this invention.

For example, a problem found in the paper type "wet laid" when used in the manufacture of the absorption sheet 9 is derived from the fact of this being comprised of oriented fibers which hamper up to a certain degree the liquid absorption and distribution by the absorbing core 8, thus increasing the absorption time of liquid by the absorbing core 8, as well as the probability of occurrence of saturation points in said core 8. Thus, leakage can take place in the sanitary absorbent article.

Another problem found in the paper type "wet laid" is that the surface thereof presents a certain degree of impermeability by virtue of the manufacture process, thus hampering its application in manufacturing the absorption sheet 9. Still, a further problem of the paper type "wet laid" is that this material presents certain rigidity, causing discomfort for the user of the absorbent 1, being, therefore, inadvisable the use of this paper in sanitary absorbent articles 1 without the proper preparation.

Thus, in order to solve the problems resulting from the use of the paper "wet laid" above mentioned, it is envisaged a method of manufacture for the article 1 which preview steps of embossing and perforation of the absorbing core 8, making viable the use of this kind of paper.

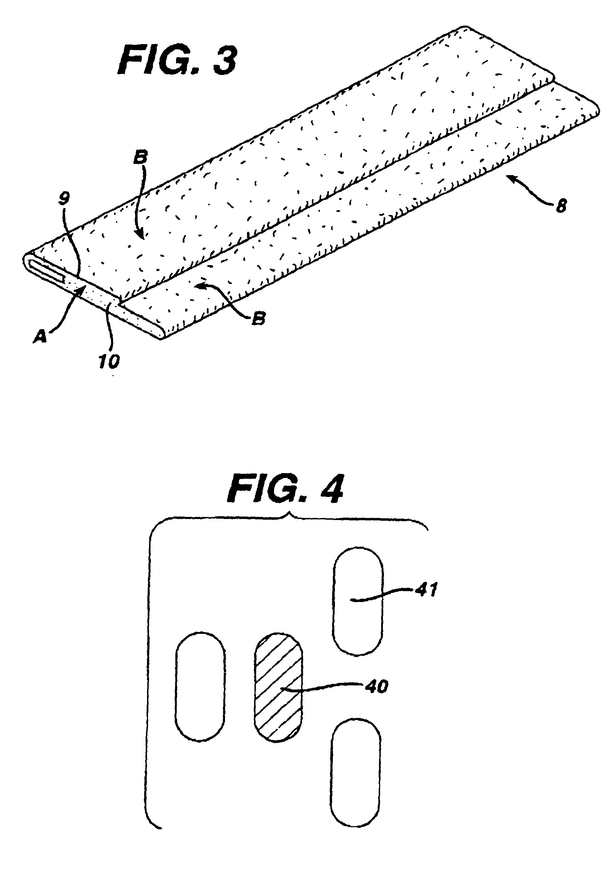

As can be seen in figure 3, the absorption sheet 9 is divided into three portions: one central A and two lateral B. The central portion A comprises a longitudinal central band of approximately 1/2 of the width of sheet 9. The lateral portions B are formed by the longitudinal sides of the sheet 9.

For manufacturing the absorbing core 8 it is envisaged, initially, a step of jetting adhesive on an inner surface of the absorption sheet 9. The process of applying adhesive on the absorption sheet 9 is also known as "spray hot melt", which uses a thermoplastic adhesive material.

Preferably, the amount of adhesive material to be applied on the inner surface of the absorption sheet 9 is in the range of 0.01 g to 1.00 g per 100 cm², most preferably, 0.30 g to 0.40 g per 100 cm². Upon the adhesive layer is applied a layer of superabsorbent material 10, known in the art itself and in the form of granules, which preferably are distributed relatively uniformly upon the adhesive layer.

The choice of a particular superabsorbent for the product has been found to provide an important role in fluid handling performance. Preferred superabsorbents have absorbency under load (AUL) value of at least about 24 ml saline per gram of superabsorbent. The test for AUL is defined in U.S. Patent 5,147,343 issued September 15, 1992 to Kellenberger. The high values of AUL values minimize the potential for the superabsorbent to gel block, especially in constructions having a wet laid paper laminate, where the amount of fibrous material available to aid in fluid distribution wicking is limited. In U.S. Patent No. 5,562,646 (issued October 8, 1996 to Goldman) additional properties which would allow the superabsorbent to perform well in such a construction are disclosed. These are the porosity, the performance under pressure, and the Saline Flow Conductivity. Relatively high values of these properties will allow the superabsorbent layer to acquire and transport fluid at the rates needed for good performance, even without any contribution from the wet laid material. Preferred values are: porosity greater than 0.15, performance under pressure of at least 23 g/g under a confining pressure of 0.7 psi and a saline flow conductivity of at least 30X10⁻⁷ cm³ sec/gr.

In the preferred embodiment, after application of the superabsorbent, the absorption sheet 9 is bent so that both lateral portions B close on the central portion A, covering the layer of superabsorbent material 10 adhered to the internal surface, thus forming the layer of superabsorbent material 10 protected by said absorption sheet 9.

Preferably, the fold of the absorption sheet 9, seen in profile, is substantially "C-shaped" (see figure 3). Alternative embodiments include a "G- shaped" profile or may even assume different profiles that are substantially equivalent. As can be seen in figure 3, the ends of the side portions B meet when they are bent, being dispensed to the fixation from one end to another, since the adhesive itself keeps the side portions B closed on the portion A.

Preferably, the face of the core 8 where the sides B of the absorption sheet 9 meet is positioned in such a way that it faces the side of the impervious lower layer 3 of the absorbent 1.

In the preferred embodiment, once the absorption sheet 9 is bent, the absorbing core 8 goes through an embossing and perforation operation.

The embossing of the material is a known technique and it can be applied in form planar or in cut, or in another equivalent form. Such technique increases the contact surface of the absorption sheet 9 and, therefore, increases the absorption power of the absorbing core 8.

The embossing is applied through two embossing rolls, an upper one and a lower one, each of them having a surface with a relief formed by teeth with specific format. Such relief will be transferred to the material to be embossed, that is, in the present case it will be transferred to the absorbing core 8 during the embossing. Still as known in the art, a measure that must be considered in the embossing process is called depth band. This measure reveals how much the extreme edge of a tooth or relief of a roll overcomes the extreme edge of the relief tooth of another roll.

The kind of embossing preferably applied to the absorbing core is known in the art as "side x side centered". As can be seen in figure 4, in this kind of embossing, the upper roll applies a demarcation 40 positioned among three demarcations 41 applied by the lower roll. Preferably, said depth band used in this invention is between 0.030 to 0.040 inches (0.762 to 1.016 mm), being most preferably 0.035 inches (0.889 mm). Preferably, the embossing velocity is 30 feet per minute (9.144 meters per minute).

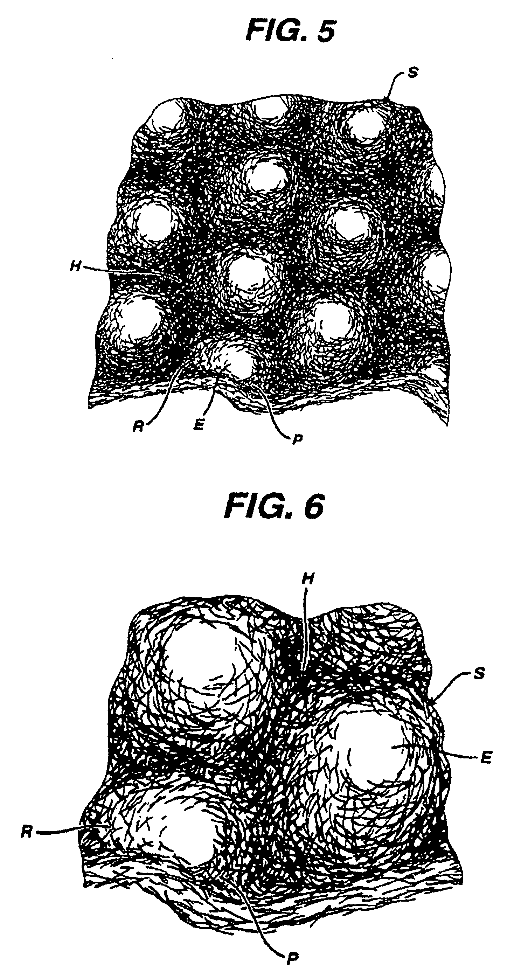

As illustrated in Figs. 5 and 6, this embossing applied to the absorbing core 8 modifies the surface of the absorption sheet 9. This results in a uniform standard of elevations, E, which project from said surface of the sheet 9. Each of these elevations, E, has a substantially triangular profile, where one of the sides, R, has a gradual elevation in slope and the opposite side, P, has an elevation more abrupt forming a wall substantially perpendicular to the surface of the sheet 9.

The kind of embossing above described causes the appearance of a certain number of holes, H, on surface, S, of the absorbing core 8, thus enabling a suitable distribution of the menstrual fluid in addition to a greater absorption velocity, even using the paper type "wet laid" in manufacturing the core 8.

Said holes, H, are created at the point where the base of the wall, P, meets the surfce of the sheet 9, since in this region occurs the elevation of the surface pressure of the sheet 9 paper, and causing the rupture of its fibers, causing small tearing in the material and the appearance of said holes.

The existence of the superabsorbent granules 10 contained inside the absorbing core 8 still contributes to the appearance of said holes, since such granules chafe with the absorption sheet 9 causing the holes on the paper.

Preferably, the application of embossing to the absorbing core 8 results in walls, P, that are positioned longitudinally in relation to the longitudinal axis of the sanitary absorbent article 1. Such positioning of the walls, P, will cause the menstrual fluid to spread more longitudinally along the absorbing core 8, being drained by the superabsorbent material 10 through the holes.

The presence of the superabsorbent material 10 in the edge of the hole still contributes to the best absorption of the menstrual fluid, because the direct contact of this material 10 gives rise to the faster absorption of the fluid.

In the preferred embodiment the distribution of the elevations on the surface of the absorbing core 8 are in the following form: each cm² has about 8 to 15 elevations on its upper surface and the same amount of elevations on its lower surface. Most preferably, each cm² has 12 elevations on the upper surface and 12 elevations on the lower surface of the absorbing core 8.

In the preferred embodiment, the distribution of holes is about 2 to 15 holes per cm² on the upper and lower surfaces of the core 8. Most preferably, the distribution of the holes is about 6 holes per cm² in said upper and lower layers of the core 8.

Additional advantages are attained from the method of manufacture above described, in addition to the substantial economy in cost of the material for manufacturing the absorbent 1.

The perforation of the absorption sheet 9 of the absorbing core 8, as already described, causes the superabsorbent material 10 to be partially exposed, giving rise to the liquid, which reaches said absorbing core 8, to be directly absorbed by the superabsorbent material 10, drying quickly the upper pervious layer 2 as well as the transfer layer 7 and providing a greater comfort to the user of the sanitary absorbent article 1.
The perforation of the absorbing core 8 offers an improved absorption and distribution of the liquid, reducing the possibility of leakage of the article 1, in addition to improving the flexibility of the "wet laid" paper, since the perforation breaks the oriented fibers of this material.

Further, the perforation breaks the impermeability found on the surface of the paper "wet laid", offering an absorbing core 8 with improved absorption of liquid.

A further advantage of this invention is that the embossing, in conjunction with the perforation, results in an absorbing core 8 and accordingly a sanitary absorbent article 1 with slightly greater thickness, when compared to the sanitary absorbent articles of the state of the art, offering a greater sensation of safety to the user.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

## Claims

1. A sanitary absorbent article comprising:
an upper layer pervious to liquid;
a lower layer impervious to liquid;
a transfer layer; and,
an absorbing core having an upper part and a lower part, said core being formed from an absorption sheet and a superabsorbent material adhered to an inner surface of the sheet, said sheet consisting essentially of a wet laid paper and comprising two opposite longitudinal sides, each said longitudinal side having been bent onto the inner surface, and wherein the absorbing core is embossed and perforated.

2. The absorbent article according to claim 1, wherein the absorbing core comprises a plurality of elevations on its surface, the elevations having an essentially triangular profile wherein one side has a gradual elevation and one opposite side has an abrupt elevation, the side having an abrupt elevation being positioned essentially parallel in relation to the longitudinal axis of the absorbing core.

3. The absorbent article according to claim 2, wherein the absorbent core comprises 8 to 15 elevations per cm² both in the upper part and in the lower part.

4. The absorbent article according to claim 1, wherein the absorbing core comprises 2 to 15 perforations per cm² both in the upper part and in the lower part.

5. The absorbent article according to claim 1, wherein the superabsorbent material has an absorbency under load value of at least about 24 ml saline per gram of superabsorbent material.

6. The absorbent article according to claim 1, wherein the superabsorbent material has a porosity of at least about 0.15.

7. The absorbent article according to claim 1, wherein the superabsorbent material has a Performance under Pressure capacity value of at least about 23 g/g under a confining pressure of 0.7 psi.

8. The absorbent article according to claim 1, wherein the superabsorbent material has a Saline Flow Conductivity value of at least about 30x10⁻⁷ cm³ sec/g.

9. An absorbent core for use in a sanitary article, said core having an upper part and a lower part and comprising an absorption sheet and superabsorbent material, the superabsorbent material adhered to an inner surface of the sheet, said sheet and said superabsorbent material primarily forming said core, said sheet consisting essentially of wet laid paper laid paper and comprising two opposite longitudinal sides, each said longitudinal side having been bent onto the inner surface, and wherein the absorbent core is embossed and perforated.

10. The absorbent core according to claim 9, wherein the absorbent core comprises a plurality of elevations its surface, the elevations having an essentially triangular profile wherein one side has a gradual elevation and one opposite side has an abrupt elevation, the side having an abrupt elevation being positioned essentially parallel in relation to the longitudinal axis of the absorbing core.

11. The absorbent core according to claim 10, wherein the absorbent core comprises 8 to 15 elevations per cm² both in the upper part and in the lower part.

12. The absorbent core according to claim 9, wherein the absorbent core comprises 2 to 15 perforations per cm² both in the upper part and in the lower part.

13. Method of manufacturing an absorbing core for use in a sanitary absorbent article, said method comprising the steps of:
- jetting adhesive material on a paper sheet of the type "wet laid;
- applying superabsorbent material onto the adhesive material;
- bending the paper sheet thereby forming said absorbing core; and,
- embossing and perforating the absorbing core.

14. The method according to claim 13, wherein said embossing step creates a plurality of elevations on the surface of the absorbing core, the elevations having an essentially triangular profile wherein one side has a gradual elevation and one opposite side has an abrupt elevation, the side having an abrupt elevation being positioned essentially parallel in relation to the longitudinal axis of the absorbing core.

## Patentansprüche

1. Absorbierender Hygieneartikel, umfassend:
eine flüssigkeitsdurchlässige obere Schicht;
eine flüssigkeitsundurchlässige untere Schicht;
eine Transferschicht; und
einen absorbierenden Kern mit einem oberen Teil und einem unteren Teil,
wobei der Kern aus einer Absorptionslage und einem superabsorbierenden Material, das an der inneren Oberfläche der Lage haftet, ausgebildet ist und wobei die Lage im wesentlichen ein feucht aufgelegtes Papier enthält und zwei gegenüberliegende longitudinale Seiten umfaßt und jede longitudinale Seite auf die innere Oberfläche gebogen ist, **dadurch gekennzeichnet, daß** der absorbierende Kern geprägt und perforiert ist.

2. Absorbierender Artikel nach Anspruch 1, **dadurch gekennzeichnet, daß** der absorbierende Kern eine Vielzahl von Erhöhungen auf seiner Oberfläche umfaßt, daß die Erhöhungen ein im wesentlichen dreieckiges Profil aufweisen, wobei eine Seite eine graduelle Erhöhung und eine gegenüberliegende Seite eine abrupte Erhöhung aufweist und die Seite mit der abrupten Erhöhung im wesentlichen parallel im Verhältnis zu der longitudinalen Achse des absorbierenden Kerns positioniert ist.

3. Absorbierender Artikel nach Anspruch 2, **dadurch gekennzeichnet, daß** der absorbierende Kern 8 bis 15 Erhöhungen pro cm² sowohl in dem oberen Teil als auch in dem unteren Teil umfaßt.

4. Absorbierender Artikel nach Anspruch 1, **dadurch gekennzeichnet, daß** der absorbierende Kern 2 bis 15 Perforierungen pro cm² sowohl in dem oberen Teil als auch in dem unteren Teil umfaßt.

5. Absorbierender Artikel nach Anspruch 1, **dadurch gekennzeichnet, daß** das superabsorbierende Material einen Absorptionswert unter Belastung von mindestens zirka 24 ml Salzlösung pro Gramm superabsorbierenden Materials aufweist.

6. Absorbierender Artikel nach Anspruch 1, **dadurch gekennzeichnet, daß** das superabsorbierende Material eine Porösität von mindestens zirka 0,15 aufweist.

7. Absorbierender Artikel nach Anspruch 1, **dadurch gekennzeichnet, daß** das superabsorbierende Material einen Leistungs-unter-Druck-Kapazitätswert von mindestens zirka 23 Gew.-% unter einem begrenzenden Druck von 0,7 psi aufweist.

8. Absorbierender Artikel nach Anspruch 1, **dadurch gekennzeichnet, daß** das superabsorbierende Material einen Salzlösungsfluß-Leitfähigkeitswert von mindestens zirka 30x10⁻⁷ cm³ Sek./g aufweist.

9. Absorbierender Kern zum Einsatz in einem Hygieneartikel, bei dem der Kern einen oberen Teil und einen unteren Teil aufweist und eine Absorptionslage und superabsorbierendes Material umfaßt, wobei das superabsorbierende Material an einer inneren Oberfläche der Lage haftet und die Lage und das superabsorbierende Material im wesentlichen den Kern ausbilden und wobei die Lage im wesentlichen feucht aufgelegtes Papier enthält und zwei gegenüberliegende longitudinale Seiten umfaßt und jede longitudinale Seite auf die innere Oberfläche gebogen ist, **dadurch gekennzeichnet, daß** der absorbierende Kern geprägt und perforiert ist.

10. Absorbierender Kern nach Anspruch 9, **dadurch gekennzeichnet, daß** der absorbierende Kern eine Vielzahl von Erhöhungen auf seiner Oberfläche aufweist, daß die Erhöhungen ein im wesentlichen dreieckiges Profil haben, wobei eine Seite eine graduelle Erhöhung und eine gegenüberliegende Seite eine abrupte Erhöhung aufweist und die Seite mit der abrupten Erhöhung im wesentlichen parallel im Verhältnis zur longitudinalen Achse des absorbierenden Kerns positioniert ist.

11. Absorbierender Kern nach Anspruch 10, **dadurch gekennzeichnet, daß** der absorbierende Kern 8 bis 15 Erhöhungen pro cm² sowohl in dem oberen Teil als auch in dem unteren Teil umfaßt.

12. Absorbierender Kern nach Anspruch 9, **dadurch gekennzeichnet, daß** der absorbierende Kern 2 bis 15 Perforierungen pro cm² sowohl in dem oberen Teil als auch in dem unteren Teil umfaßt.

13. Verfahren zur Herstellung eines absorbierenden Kerns zum Einsatz in einem absorbierenden Hygieneartikel, wobei das Verfahren die folgenden Schritte umfaßt:
- Aufspritzen eines haftenden Materials auf eine Lage Papier des Typs "feucht aufgelegt";
- Aufbringen des superabsorbierenden Materials auf das haftende Material;
- Biegen der Papierlage, wodurch der absorbierende Kern ausgebildet wird; und
- Prägen und Perforieren des absorbierenden Kerns.

14. Verfahren nach Anspruch 13, **dadurch gekennzeichnet, daß** der Schritt des Prägens eine Vielzahl von Erhöhungen auf der Oberfläche des absorbierenden Kerns ausbildet, daß die Erhöhungen ein im wesentlichen dreieckiges Profil aufweisen, wobei eine Seite eine graduelle Erhöhung und eine gegenüberliegende Seite eine abrupte Erhöhung aufweist und die Seite mit der abrupten Erhöhung im wesentlichen parallel im Verhältnis zu der longitudinalen Achse des absorbierenden Kerns positioniert ist.

## Revendications

1. Article hygiénique absorbant comprenant :
◆ une couche supérieure perméable au liquide ;
◆ une couche inférieure imperméable au liquide ;
◆ une couche de transfert ; et
◆ un noyau absorbant possédant une partie supérieure et une partie inférieure, ledit noyau étant formé d'une feuille d'absorption et d'un matériau super-absorbant collé à une surface interne de la feuille, ladite feuille consistant essentiellement en un papier vergé humide et comprenant deux côtés longitudinaux opposés, chacun desdits côtés longitudinaux ayant été plié sur la surface interne, et dans lequel le noyau absorbant est gaufré et perforé.

2. Article absorbant selon la revendication 1, dans lequel le noyau absorbant comprend une pluralité de reliefs sur sa surface, les reliefs ayant un profil sensiblement triangulaire dans lequel un côté présente un relief progressif et un côté opposé présente un relief abrupte, le côté présentant un relief abrupte étant positionné de manière sensiblement parallèle par rapport à l'axe longitudinal du noyau absorbant.

3. Article absorbant selon la revendication 2, dans lequel le noyau absorbant comprend 8 à 15 reliefs par cm² à la fois dans la partie supérieure et dans la partie inférieure.

4. Article absorbant selon la revendication 1, dans lequel le noyau absorbant comprend 2 à 15 perforations par cm² à la fois dans la partie supérieure et dans la partie inférieure.

5. Article absorbant selon la revendication 1, dans lequel le matériau super-absorbant présente une valeur dé capacité d'absorption sous charge d'au moins environ 24 ml de solution saline par gramme de matériau super-absorbant.

6. Article absorbant selon la revendication 1, dans lequel le matériau super-absorbant présente une porosité d'au moins environ 0,15.

7. Article absorbant selon la revendication 1, dans lequel le matériau super-absorbant présente une valeur de capacité de performance sous pression d'au moins environ 23 g/g sous une pression de confinement de 0,7 psi.

8. Article absorbant selon la revendication 1, dans lequel le matériau super-absorbant possède une valeur de conductivité de flux salin d'au moins environ 30x10⁻⁷ cm³ sec/g.

9. Noyau absorbant pour une utilisation dans un article hygiénique, ledit noyau présentant une partie supérieure et une partie inférieure et comprenant une feuille d'absorption et un matériau super-absorbant, le matériau super-absorbant collé à une surface interne de la feuille, ladite feuille et ledit matériau super-absorbant formant principalement ledit noyau, ladite feuille consistant essentiellement en un papier vergé humide et comprenant deux côtés longitudinaux opposés, chacun desdits côtés longitudinaux ayant été plié sur la surface interne, et dans lequel le noyau absorbant est gaufré et perforé.

10. Noyau absorbant selon la revendication 9, dans lequel le noyau absorbant comprend une pluralité de reliefs sur sa surface, les reliefs ayant un profil sensiblement triangulaire dans lequel un côté présente un relief progressif et un côté opposé présente un relief abrupt, le côté présentant un relief abrupt étant positionné de manière sensiblement parallèle par rapport à l'axe longitudinal du noyau absorbant.

11. Noyau absorbant selon la revendication 10, dans lequel le noyau absorbant comprend 8 à 15 reliefs par cm² à la fois dans la partie supérieure et dans la partie inférieure.

12. Noyau absorbant selon la revendication 9, dans lequel le noyau absorbant comprend 2 à 15 perforations par cm² à la fois dans la partie supérieure et dans la partie inférieure.

13. Procédé de fabrication d'un noyau absorbant pour utilisation dans un article hygiénique absorbant, ledit procédé comprenant les étapes consistant à :
◆ projeter un matériau adhésif sur une feuille de papier de type " vergé humide " ;
◆ appliquer le matériau super-absorbant sur le matériau adhésif ;
◆ plier la feuille de papier formant ainsi ledit noyau absorbant ; et
◆ gaufrer et perforer le noyau absorbant.

14. Procédé selon la revendication 13, dans lequel ladite étape de gaufrage crée une pluralité de reliefs sur la surface du noyau absorbant, les reliefs ayant un profil sensiblement triangulaire dans lequel un côté possède un relief progressif et un côté opposé présente un relief abrupt, le côté présentant un relief abrupt étant positionné de manière sensiblement parallèle par rapport à l'axe longitudinal du noyau absorbant.
